# EUROPEAN PATENT APPLICATION

(11) **EP 4 410 265 A1**
(43) Date of publication of application: **07.08.2024**
(21) Application number: 21923595.9
(22) Date of filing: 09.11.2021
(51) Int. Cl.: A61K 8/06, A61K 8/02, A61K 8/34, A61K 8/20, A61K 8/92, A61Q 19/00

(54) **WATER-IN-OIL SOLID COSMETIC COMPOSITION HAVING APPROPRIATE RATIO OF AQUEOUS PHASE AND OIL PHASE**

(30) Priority: 28.09.2021 KR 20210128261
(71) Applicant: Cosmax, Inc., Hwaseong-si, Gyeonggi-do 18622 (KR)
(72) Inventor: PARK, Hae Ryung, Seoul 06001 (KR); LIM, Hyeon Joo, Gyeonggi-do 13523 (KR); JI, Jin Goo, Gyeonggi-do 16363 (KR); PARK, Myeong Sam, Seoul 04987 (KR)
(74) Representative: Plougmann Vingtoft a/s
(86) International application number: PCT/KR2021/016207
(87) International publication number: WO 2023/054797

(57) **Abstract**

The present invention relates to a water-in-oil solid cosmetic composition stably containing an aqueous phase and an oil phase, and the water-in-oil solid cosmetic composition can be implemented as a stable formulation by containing the aqueous phase and the oil phase at an appropriate ratio, and has effects of causing no skin troubles, having reduced stickiness, and providing an improved moisturizing feeling by containing a large amount of water-soluble ingredients instead of silicone and other non-aqueous raw materials in the conventional art.

## Description

### Technical Field

This application claims priority to Korean Patent Application No. 10-2021-0128261 filed in the Korean Intellectual Property Office on 28 September 2021, the disclosure of which is incorporated herein by reference.

The present disclosure relates to a water-in-oil solid cosmetic composition having an improved moisturizing effect by stably containing an aqueous phase and an oil phase at an appropriate ratio.

### Background Art

A conventional solid cosmetic composition refers to a formulation obtained by solidifying an oil with excellent moisturizing ability and is widely used as a moisturizer due to the advantage of not flowing down owing to high hardness thereof. However, typical solid cosmetic compositions indicate an oil-dispersible formulation containing only oil-phase raw materials and do not usually contain a water-soluble ingredient.

In many cases, oil-dispersible solid cosmetic formulations are composed of only silicone and other non-aqueous raw materials, and the application of these ingredients to oily skin may block pores of the skin to cause severe troubles. Moreover, the oil-dispersible solid cosmetic compositions are sticky for a long time rather than directly penetrating the skin upon application, causing discomfort in life, and the combined use of the oil-dispersible cosmetic compositions with other cosmetic compositions may cause the separation of applied materials from the skin.

There is an urgent need to develop a water-in-oil solid cosmetic composition having an appropriate ratio between an aqueous phase and an oil phase and being capable of causing no skin troubles, reducing stickiness, and providing an improved moisturizing feeling.

### Disclosure of Invention

### Technical Problem

The present inventors have made intensive research efforts to develop a water-in-oil solid cosmetic composition having an appropriate ratio between an aqueous phase and an oil phase. As a result, the present inventors confirmed that the water-in-oil solid cosmetic composition containing an aqueous phase and an oil phase at an appropriate ratio has excellent effects of reducing stickiness, providing an improved moisturizing feeling, and causing no skin troubles compared with existing oil-dispersible solid cosmetic compositions composed of only silicone and other non-aqueous raw materials.

Accordingly, an aspect of the present disclosure is to provide a water-in-oil solid cosmetic composition containing an aqueous phase and an oil phase.

### Solution to Problem

Hereinafter, the present disclosure will be described in more detail.

In accordance with an aspect of the present disclosure, there is provided a water-in-oil (W/O) solid cosmetic composition comprising an aqueous phase and an oil phase.

In the present disclosure, the weight ratio between the aqueous phase and the oil phase may be 4:6 to 6:4, for example, 5:5, but is not limited thereto.

In the present disclosure, the aqueous phase may be contained, relative to the total cosmetic composition, in a content of 30.0 to 50.0 wt%, 30.0 to 47.5 wt%, 35.0 to 50.0 wt%, 35.0 to 47.5 wt%, 40.0 to 50.0 wt%, 40.0 to 47.5 wt%, 45.0 to 50.0 wt%, or 45.0 to 47.5 wt%, and for example, 47.1 wt%, but is not limited thereto.

In the present disclosure, the aqueous phase may be at least one selected from the group consisting of a polyol, a pH adjuster, and a solvent, but is not limited thereto.

In the present disclosure, the polyol may be at least one selected from the group consisting of glycerin, butylene glycol, pentylene glycol, 1,2-hexanediol, propanediol, methyl propanediol, and dipropylene glycol.

In the present disclosure, the polyol may be contained, relative to the total cosmetic composition, in a content of 5.0 to 15.0 wt%, 5.0 to 13.0 wt%, 5.0 to 11.0 wt%, 7.0 to 15.0 wt%, 7.0 to 13.0 wt%, 7.0 to 11.0 wt%, 9.0 to 15.0 wt%, 9.0 to 13.0 wt%, 9.0 to 11.0 wt%, 11.0 to 15.0 wt%, or 11.0 to 13.0 wt%, and for example, 11.0 wt%, but is not limited thereto.

In the present disclosure, the pH adjuster may be sodium chloride, but is not limited thereto.

In the present disclosure, the pH adjuster may be contained, relative to the total cosmetic composition, in a content of 0.3 to 1.0 wt%, 0.1 to 0.8 wt%, 0.1 to 0.6 wt%, 0.3 to 1.0 wt%, 0.3 to 0.8 wt%, 0.5 to 1.0 wt%, 0.5 to 0.8 wt%, or 0.5 to 0.6 wt%, and for example, 0.5 wt%, but is not limited thereto.

In the present disclosure, the oil phase may be at least one selected from the group consisting of a surfactant, a wax, an oil, and a vegetable butter, but is not limited thereto.

In the present disclosure, the surfactant may be at least one selected from the group consisting of polyglyceryl-4 isostearate, polyglyceryl-3 polyricinoleate, disteardimonium hectorite, and sorbitan isostearate, but is not limited thereto.

In the present disclosure, the surfactant may be contained, relative to the total cosmetic composition, in a content of 5.0 to 10.0 wt%, 5.0 to 8.0 wt%, 7.0 to 10.0 wt%, or 7.0 to 8.0 wt%, and for example, 7.0 wt%, but is not limited thereto.

In the present disclosure, the wax may be at least one selected from the group consisting of ozokerite, polyethylene, synthetic wax, and microcrystalline wax, but is not limited thereto.

In the present disclosure, the wax was may be contained, relative to the total cosmetic composition, in a content of 12.0 to 18.0 wt%, 12.0 to 16.0 wt%, 14.0 to 18.0 wt%, or 14.0 to 16.0 wt%, and for example, 15.0 wt%, but is not limited thereto.

In the present disclosure, the oil may be at least one selected from the group consisting of coco-caprylate/caprate, isononyl isononanoate, hydrogenated coconut oil, diisostearyl malate, Camellia japonica seed oil, apple seed oil, stearyl heptanoate, stearyl caprylate, and dipentaerythrityl hexahydroxystearate/hexastearate/hexarosinate, but is not limited thereto.

In the present disclosure, the oil may be contained, relative to the total cosmetic composition, in a content of 15.0 to 25.0 wt%, 15.0 to 23.0 wt%, 17.0 to 25.0 wt%, 17.0 to 23.0 wt%, 20.0 to 25.0 wt%, or 20.00 to 23.0 wt%, and for example, 20.0 wt%, but is not limited thereto.

In the present disclosure, the vegetable butter may be at least one selected from the group consisting of shea butter, cupuacu seed butter, mango seed butter, and cacao seed butter, but is not limited thereto.

In the present disclosure, the vegetable butter may be contained, relative to the total cosmetic composition, in a content of 0.5 to 1.5 wt%, 0.5 to 1.3 wt%, 0.5 to 1.0 wt%, 0.7 to 1.5 wt%, 0.7 to 1.3 wt%, 0.9 to 1.5 wt%, 0.9 to 1.3 wt%, or 0.9 to 1.0 wt%, and for example, 1.0 wt%, but is not limited thereto.

In the present disclosure, the water-in-oil solid cosmetic composition may contain a powder, but is not limited thereto.

In the present disclosure, the powder may be at least one selected from the group consisting of silica and a styrene/acrylate copolymer.

In the present disclosure, the powder may be contained, relative to the total cosmetic composition, in a content of 1.0 to 5.0 wt%, 1.0 to 4.5 wt%, 1.0 to 4.0 wt%, 1.0 to 3.5 wt%, 1.0 to 3.0 wt%, 1.0 to 2.5 wt%, 1.0 to 2.0 wt%, 1.5 to 5.0 wt%, 1.5 to 4.5 wt%, 1.5 to 4.0 wt%, 1.5 to 3.5 wt%, 1.5 to 3.0 wt%, 1.5 to 2.5 wt%, or 1.5 to 2.0 wt%, and for example, 2.0 wt%, but is not limited thereto.

### Advantageous Effects of Invention

The present disclosure is directed to a water-in-oil solid cosmetic composition stably containing an aqueous phase and an oil phase, and the water-in-oil solid cosmetic composition can be implemented as a stable formulation by containing the aqueous phase and the oil phase at an appropriate ratio, and has effects of causing no skin troubles, having reduced stickiness, and providing an improved moisturizing feeling by containing a large amount of water-soluble ingredients instead of silicone and other non-aqueous raw materials in the conventional art.

### Brief Description of Drawings

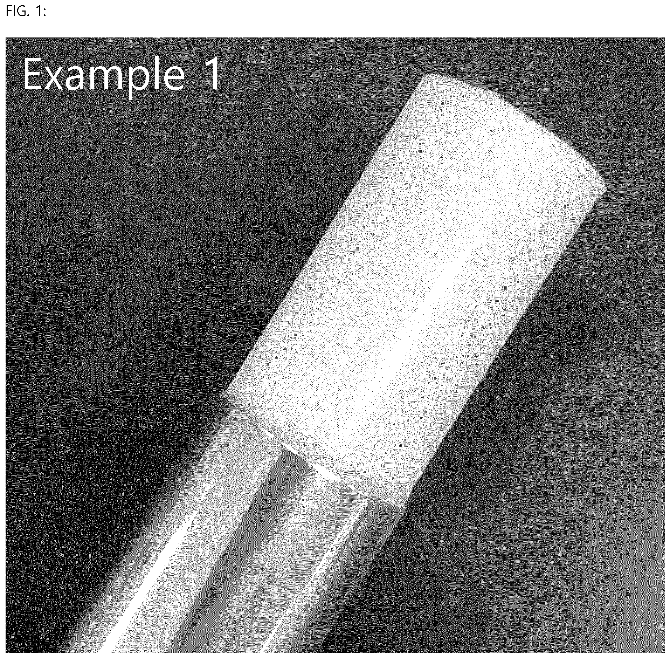

FIG. 1 is a picture showing a formulation of Example 1 according to a test example of the present disclosure.

### Best Mode for Carrying out the Invention

The present disclosure relates to a water-in-oil (W/O) solid cosmetic composition comprising an aqueous phase and an oil phase.

### Mode for Carrying out the Invention

Hereinafter, the present disclosure will be described in more detail by the following examples. However, these exemplary embodiments are used only for illustration, and the scope of the present disclosure is not limited by these exemplary embodiments.

### Preparative Example: Preparation of Example and Comparative Examples

Water-in-oil solid cosmetic compositions of Example 1 and Comparative Examples 1 and 2 were prepared according to the respective ingredients and contents thereof shown in Table 1 below.

**TABLE 1**

| N O | Classifica tion | Classifica tion | Ingredient | Examp le 1 | Compara tive Example 1 | Compara tive Example 2 |
|---|---|---|---|---|---|---|
| 1 | Aqueous phase | Polyol | Glycerin | 2.0 | 20.0 | 5.0 |
| 2 | | | butylene glycol | 9.0 | 15.0 | 5.0 |
| 3 | | | pentylene glycol | 1.5 | 1.5 | 1.5 |
| 4 | | | 1,2-hexanediol | 0.5 | 0.5 | 0.5 |
| 5 | | pH adjuster | sodium chloride | 0.5 | 0.5 | 0.5 |
| 6 | | Solvent | Purified water | 33.6 | 29.6 | 16.6 |
| 7 | Oil phase | Surfactant | polyglyceryl-4 isostearate | 3.8 | 3.8 | 3.8 |
| 8 | | | polyglyceryl-3 polyricinoleate | 1.6 | 1.6 | 1.6 |
| 9 | | | disteardimonium hectorite | 0.9 | 0.9 | 0.9 |
| 10 | | | sorbitan isostearate | 0.6 | 0.6 | 0.6 |
| 1 1 | | Wax | ozokerite | 12.0 | 12.0 | 12.0 |
| 1 2 | | | polyethylene | 1.0 | 1.0 | 1.0 |
| 1 3 | | | synthetic wax | 1.0 | 1.0 | 1.0 |
| 1 4 | | | microcrystallin e wax | 1.0 | 1.0 | 1.0 |
| 1 5 | | Oil | coco-caprylate/capra te | 15.0 | 3.0 | 25.0 |
| 1 6 | | | isononyl isononanoate | 9.0 | 3.0 | 15.0 |
| 1 7 | | | hydrogenated coconut oil | 3.0 | 1.0 | 5.0 |
| 1 8 | | | diisostearyl malate | 1.0 | 1.0 | 1.0 |
| 1 9 | | Vegetable butter | shea butter | 1.0 | 1.0 | 1.0 |
| 2 0 | Film former | Powder | silica | 1.0 | 1.0 | 1.0 |
| 2 1 | | | vinyl dimethicone/met hicone silsesquioxane crosspolymer | 1.0 | 1.0 | 1.0 |
| Total (unit: wt%) | | | | 100.0 | 100.0 | 100.0 |

Specifically, the respective raw materials shown in Table 1 were weighed in beakers, and then the surfactants of Nos. 7 to 10, the waxes, oils, and vegetable butters of Nos. 11 to 19, which correspond to an oil phase, were completely dissolved while warmed to 80°C. Thereafter, the mixtures were placed in a homomixer and stirred at 3500 rpm for 3 minutes.

Then, Nos. 1 to 6, which correspond to an aqueous phase, were warmed to 80°C to confirm the complete dissolution thereof, and then the mixtures were placed in a homomixer and stirred at 3500 rpm for 5 minutes. Then, Nos. 20 and 21, which are powders, were individually added, followed by stirring at 3500 rpm for 3 minutes. Then, bubbles were removed to prepare Example 1 and Comparative Examples 1 and 2.

### Test Example 1: Evaluation of formulation stability

Example 1 and Comparative Example 1 were evaluated for formulation stability under conditions of 4°C, room temperature, 37°C, 45°C, and cycling (the storage temperature was changed to -4°C three times every 24 hours) for 1 day, 5 days, 1 week, 2 weeks, and 4 weeks, and the results are shown in Table 2 below. The evaluation criteria were as follows.

### <Evaluation criteria>

Ⓞ; stable
△; slight unstable
X; unstable

**TABLE 2**

| | | Example 1 | Comparative Example 1 |
|---|---|---|---|
| 1 Days | 4°C | ⊚ | ⊚ |
| | Room temperature | ⊚ | ⊚ |
| | 37 °C | ⊚ | ⊚ |
| | 45 °C | ⊚ | ⊚ |
| | Cycle | ⊚ | ⊚ |
| 5 Days | 4 °C | ⊚ | ⊚ |
| | Room temperature | ⊚ | ⊚ |
| | 37 °C | ⊚ | ⊚ |
| | 45 °C | ⊚ | Δ |
| | Cycle | ⊚ | Δ |
| 1 Week | 4 °C | ⊚ | ⊚ |
| | Room temperature | ⊚ | ⊚ |
| | 37 °C | ⊚ | Δ |
| | 45 °C | ⊚ | Δ |
| | Cycle | ⊚ | X |
| 2 Weeks | 4 °C | ⊚ | ⊚ |
| | Room temperature | ⊚ | ⊚ |
| | 37 °C | ⊚ | X |
| | 45 °C | ⊚ | X |
| | Cycle | ⊚ | X |
| 4 Weeks | 4 °C | ⊚ | ⊚ |
| | 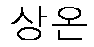 | ⊚ | ⊚ |
| | 37 °C | ⊚ | X |
| | 45 °C | ⊚ | X |
| | Cycle | ⊚ | X |

As can be confirmed in Table 2 above, as for Example 1, the formulation stability was excellent under all the temperature conditions, and the outer surface was smoothly hardened even during filling (FIG. 1).

However, as for Comparative Example 1, the formulation was unstable due to an excess aqueous phase, and after 2 weeks, the product itself tended to shrink due to moisture evaporation. As for Comparative Example 2, no solid formulation was formed since oils oozed out due to an excessive oil phase, and thus the observation of stability was not impossible.

It could be therefore confirmed that an aqueous phase and an oil phase need to be contained at a ratio of about 5:5 in order to prepare a stable water-in-oil solid cosmetic composition.

### Test Example 2: Evaluation of skin moisturization

Approximately 10 healthy male/female subjects were selected and measured for skin moisture levels. A control group applied without anything (non-application) was compared with test groups applied with Example 1 and Comparative Example 1. The test groups were measured for contents of moisture present in the skin epidermis before application initiation, immediately after application, 2 hours after application, 4 hours after application, and 8 hours after application, sequentially. The contents of moisture present in the skin epidermis were measured using a Corneometer sensor. The Corneometer is a machine that can measure and quantify the ion degree of moisture to confirm the moisturizing ability of the skin. The skin moisture levels were expressed as %, and the measurement was performed under conditions of a constant temperature of 24°C and a constant humidity (relative humidity) of 40%. The Corneometer probe was placed on the inside of the forearm, which was a site of measurement, and then the electrical conductivity quantified through the sensor was confirmed on the screen. After measurement, the sensor was wiped with a tissue, such as Kim's Wipe, and the measurement was repeated several times for different sites of measurement. The change in moisture level over time was measured, and the results are shown in Table 3.

**TABLE 3**

| (Unit: Moisture level of skin, %) | Example 1 | Comparative Example 1 |
|---|---|---|
| 0 Hrs | 100.0 | 100.0 |
| 0.5 Hrs | 185.7 | 168.9 |
| 2 Hrs | 178.6 | 154.4 |
| 4 Hrs | 155.0 | 136.8 |
| 8 Hrs | 138.2 | 123.0 |

As can be confirmed in Table 3 above, Example 1 was evaluated to have high moisture-sustaining ability since the aqueous phase and the oil phase were appropriately contained at a weight ratio of about 5:5 and thus the oils surrounded the skin after the penetration of the aqueous phase into the skin. However, Comparative Example 1 was measured to have a low moisture level in the skin epidermis due to a large amount of moisture evaporated, caused by a large amount of aqueous phase, and a high content of the powder.

It could be therefore confirmed that Example 1 containing an aqueous phase and an oil phase at an appropriate ration was excellent compared with Comparative Example 1 in the skin moisturization evaluation.

### Test Example 3: Evaluation of trans-epidermal water loss

Example 1 and Comparative Example 1 were applied to both cheeks of 5 males/females between the ages of 20 and 40 twice daily for 4 weeks, and the trans-epidermal water loss (TEWL) was measured under conditions of constant temperature and constant humidity (24°C and a relative humidity of 40%) before application, 2 weeks after application, and 4 weeks after application. The moisturizing ability of the skin was measured by calculating the amount of moisture evaporated from the skin according to the area and time and reading and quantifying the amount of evaporated moisture by using a temperature and moisture electronic sensor. The temperature and humidity corresponding to environmental conditions were maintained constant. Minimum staff, in addition to the measurer and the subjects, were left, and the flow of air was blocked as much as possible. The TEWL probe was lightly placed on the measurement sites of the subjects and allowed to form an angle of 90 degrees with respect to the ground. When the TEWL level of the skin became constant over time, the value at the time was measured for about 1 to 3 minutes, and the amount of water evaporated per hour and area (g/m²/h) was calculated. The results are shown in Table 4 below. Trans-epidermal water loss (%) = [(initial trans-epidermal water loss - trans-epidermal water loss before sameple use) / trans-epidermal water loss before sample use] × 100

**TABLE 4**

| (Unit: water loss (%)) | Example 1 | Comparative Example 1 |
|---|---|---|
| 2 Weeks after application | 40 | 90 |
| 4 Weeks after application | 50 | 85 |

As can be confirmed in Table 4 above, Example 1 showed a significantly decreased water loss compared with Comparative Example 1. At 2 weeks after application, Example 1 maintained a water loss of about 40%, but Comparative Example 1 showed an increase in water loss of about 85%. At 4 weeks after application, Example 1 maintained a water loss of about 50%, indicating superior moisturizing ability of the skin.

### Industrial Applicability

The present disclosure relates to a water-in-oil solid cosmetic composition having an improved moisturizing effect by stably containing an aqueous phase and an oil phase at an appropriate ratio.

## Claims

1. A water-in-oil (W/O) solid cosmetic composition comprising an aqueous phase and an oil phase.

2. The water-in-oil solid cosmetic composition of claim 1, wherein the weight ratio of the aqueous phase and the oil phase is 4:6 to 6:4.

3. The water-in-oil solid cosmetic composition of claim 1, wherein the aqueous phase is contained in a content of 30.0 to 50.0 wt% relative to the total cosmetic composition.

4. The water-in-oil solid cosmetic composition of claim 1, wherein the aqueous phase is at least one selected from the group consisting of a polyol, a pH adjuster, and a solvent.

5. The water-in-oil solid cosmetic composition of claim 4, wherein the polyol is at least one selected from the group consisting of glycerin, butylene glycol, pentylene glycol, 1,2-hexanediol, propanediol, methyl propanediol, and dipropylene glycol.

6. The water-in-oil solid cosmetic composition of claim 4, wherein the polyol is contained in a content of 5.0 to 15.0 wt% relative to the total cosmetic composition.

7. The water-in-oil solid cosmetic composition of claim 4, wherein the pH adjuster is sodium chloride.

8. The water-in-oil solid cosmetic composition of claim 4, wherein the pH adjuster is contained in a content of 0.3 to 1.0 wt% relative to the total cosmetic composition.

9. The water-in-oil solid cosmetic composition of claim 1, wherein the oil phase is at least one selected from the group consisting of a surfactant, a wax, an oil, and a vegetable butter.

10. The water-in-oil solid cosmetic composition of claim 9, wherein the surfactant is at least one selected from the group consisting of polyglyceryl-4 isostearate, polyglyceryl-3 polyricinoleate, disteardimonium hectorite, and sorbitan isostearate.

11. The water-in-oil solid cosmetic composition of claim 9, wherein the surfactant is contained in a content of 5.0 to 10.0 wt% relative to the total cosmetic composition.

12. The water-in-oil solid cosmetic composition of claim 9, wherein the wax is at least one selected from the group consisting of ozokerite, polyethylene, synthetic wax, and microcrystalline wax.

13. The water-in-oil solid cosmetic composition of claim 9, wherein the wax is contained in a content of 12.0 to 18.0 wt% relative to the total cosmetic composition.

14. The water-in-oil solid cosmetic composition of claim 9, wherein the oil is at least one selected from the group consisting of coco-caprylate/caprate, isononyl isononanoate, hydrogenated coconut oil, diisostearyl malate, *Camellia japonica* seed oil, apple seed oil, stearyl heptanoate, stearyl caprylate, and dipentaerythrityl hexahydroxystearate/hexastearate/hexarosinate.

15. The water-in-oil solid cosmetic composition of claim 9, wherein the oil is contained in a content of 15.0 to 25.0 wt% relative to the total cosmetic composition.

16. The water-in-oil solid cosmetic composition of claim 9, wherein the vegetable butter is at least one selected from the group consisting of shea butter, cupuacu seed butter, mango seed butter, and cacao seed butter.

17. The water-in-oil solid cosmetic composition of claim 1, wherein the water-in-oil solid cosmetic composition comprises a powder.
